(19) Europäisches Patentamt
European Patent Office
Office européen des brevets

(11) Veröffentlichungsnummer: **0 511 226 B1**

(12) **EUROPÄISCHE PATENTSCHRIFT**

(45) Veröffentlichungstag der Patentschrift: **08.03.95**

(51) Int. Cl.[6]: **C12N 1/20**

(21) Anmeldenummer: **91901288.0**

(22) Anmeldetag: **24.12.90**

(86) Internationale Anmeldenummer:
**PCT/EP90/02301**

(87) Internationale Veröffentlichungsnummer:
**WO 91/10721 (25.07.91 91/17)**

# (54) VERFAHREN ZUR HOCHZELLDICHTE-FERMENTATION VON ESCHERICHIA COLI IN EINEM RÜHRKESSELFERMENTOR.

(30) Priorität: **19.01.90 DE 4001518**

(43) Veröffentlichungstag der Anmeldung:
**04.11.92 Patentblatt 92/45**

(45) Bekanntmachung des Hinweises auf die Patenterteilung:
**08.03.95 Patentblatt 95/10**

(84) Benannte Vertragsstaaten:
**AT BE CH DE DK FR GB LI NL SE**

(56) Entgegenhaltungen:
**EP-A- 65 895**
**EP-A- 315 944**
**EP-A- 345 588**
**DE-A- 2 601 254**
**DE-A- 2 714 708**

**BIOTECHNOLOGY LETTERS, Band 11, Nr. 3, 1989; Seiten 155-160&NUM;**

(73) Patentinhaber: **Gesellschaft für Biotechnologische Forschung mbH (GBF)**
**Mascheroder Weg 1**
**D-38124 Braunschweig (DE)**

Patentinhaber: **HANS-KNÖLL-INSTITUT FÜR NATURSTOFF-FORSCHUNG E.V.**
**Beutenbergstrasse 9**
**D-07745 Jena (DE)**

(72) Erfinder: **DECKWER, Wolf-Dieter**
**Mascheroder Weg 1**
**D-3300 Braunschweig (DE)**
Erfinder: **KNORRE, Wofgang**
**Beutenbergstr. 9**
**D-6900 Jena (DE)**
Erfinder: **KORZ, Dieter**
**Mascheroder Weg 1**
**D-3300 Braunschweig (DE)**
Erfinder: **POHL, Hans, Dieter**
**Beutenbergstr. 9**
**D-6900 Jena (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

FSTA JOURNAL, 91-08, B-0215; D. RIESENBERG et al., Nr. 91042700&NUM;

BIOTECHNOLOGY, vol. 7, November 1989, New York, NY (US); L. EPPSTEIN et al., pp. 1178-1181&NUM;

Erfinder: **RIESENBERG, Dieter**
**Beutenbergstr. 9**
**D-6900 Jena (DE)**
Erfinder: **ROSS, Anton**
**Mascheroder Weg 1**
**D-3300 Braunschweig (DE)**
Erfinder: **SANDERS, Ernst**
**Mascheroder Weg 1**
**D-3300 Braunschweig (DE)**
Erfinder: **SANDERS, Ernst**
**Mascheroder Weg 1**
**D-3300 Braunschweig (DE)**
Erfinder: **SCHULZ, Volker**
**Beutenbergstr. 9**
**D-6900 Jena (DE)**

(74) Vertreter: **Boeters, Hans Dietrich, Dr. et al**
**Patentanwälte Boeters & Bauer**
**Bereiteranger 15**
**D-81541 München (DE)**

## Beschreibung

### Anwendungsgebiete der Erfindung

Die Erfindung betrifft ein Verfahren zur Hochzelldichte-Fermentation von E.-coli-Stämmen, speziell solche, die sich als Wirte für Vektoren mit rekombinanten Expressionssystemen eignen. Das Anwendungsgebiet der Erfindung liegt in jenen Industriezweigen einer Volkswirtschaft, die einen Biotechnologiesektor aufweisen und in diesen E.-coli-Stämme als Produzentenorganismen einsetzen.

### Charakteristik der bekannten technischen Lösungen

E. coli wird häufig als zellulärer Wirt für die Bildung rekombinanter DNA-Produkte verwendet. Neben einer hohen intrazeilulären Konzentration an dem gewünschten Produkt ist Die Erzieiung hoher Konzentrationen an Zellen im Fermentor eine entscheidende Vorraussetzung für eine hohe Gesamtausbeute.

Bei der Kultivierung zu hohen Zelldichten im üblichen Rührkesselfermentor wird man mit den Problemen der Wachstumsinhibition durch zu hohe Sudstratanfangskonzentrationen, dem Verbrauch essentieller Nährmedienbestandteile im Verlaufe der Kultivierung, der Bildung von inhibitorisch wirkenden Nebenprodukten des Stoffwechels und der begrenzten Kapazität der Sauerstoffzuführung konfrontiert.

Den steigenden Bedarf an Sauerstoff einer wachsenden E. coli-Kultur kann man durch verschiedene Strategien zu decken versuchen, nämlich: Steigerung der Rührerdrehzahl und der Begasungsrate, Zuführung von sauerstoffangereicherter Luft (Jung et al. 1988; Fass et al. 1989; Krüger 1989; Eppstein et al. 1989), Begasung mit reinem Sauerstoff (Bauer und Shiloach 1974; Shiloach und Bauer 1975; Bauer und White 1976; Bauer und Ziv 1976; Mori et al. 1979; Gieiser und Bauer 1981; Mitzutani et al. 1986; Bailey et al. 1987; Pan et al. 1987; Krüger 1989), Kultivierung bei niedrigen Temperaturen (Shiloach und Bauer 1975; Bauer und White 1976; Bauer und Ziv 1976) und bei erhöhtem Druck (Tsai et al. 1987).

Mit überschüssigem Sauerstoff wurde E. coli in einem Glukose-Mineralsalzmedium bis zu 16,5 g Biotrockenmasse X/l fermentiert (Reiling ei al. 1985). Krüger (1989) und Eppstein et al. (1989) erreichten 19 g X/l. Wachstum zu höheren Zelldichten erforderte eine Fed-batch-Technik, um einerseits Inhibition und Limitation von Substanzen auszuschalten und andererseits die Bildung von inhibitorischen Nebenprodukten des Stoffwechsels zu verhindern. In allen Fällen war es erforderlich, neben der C-Quelle und Ammoniak als N-Quelle und pH-Regulator auch andere Nährstoffe zu dosieren. Unter Verwendung eines Glukose-Mineralsalzmediums erzielten Eppstein et al. (1989) durch zusätzliches Nachfüttern von Salzen 39 g X/l. Fass et al. (1989) erreichten durch Dosierung von Glukoselösung, in der sich auch Magnesiumsulfat befand, zu einem vorgelegten Glukose-Mineralsalzmedium 45 g X/l. Zusätzliches Nachfüttern von Salzen führte bei Pan et al. (1987) zu 95 g X/l.

Andere Fed-batch-Fermentationen wurden in Glukose-Mineralsalzmedien durchgeführt, die vor deren Beimpfung zusätzlich Hefeextrakt oder autolysiertes Hefepulver enthielten. Ohne zusätzliches Nachfüttern während des Zellwachstums außer Glukose und Ammoniak wurden 38 g X/l erhalten (Mori et al. 1979). Zusätzliches Dosieren von Salzen ergab höhere Biomassekonzentrationen mit 47 g X/l (Bauer und White 1976), 55 g X/l (Shiloach und Bauer 1975) und 68 g X/l (Bauer und Ziv 1976). Zusätzliches Dosieren von Salzen, Spurenelementen und Vitaminen führte zu 70 g X/l (Fieschko und Ritsoh 1986). Sehr hohe Zelldichten von 110 g X/l (Cutayar und Poillon 1989) und 125 g X/l (Mori et al. 1979) konnten durch ein komplexes Nachfüttern von Salzen, Hefeextrakt und Spurenelementen zusätzlich zur üblichen Dosierung von Glukose und Ammoniak erhalten werden. Eine weitere Supplementierung eines Glukose-Mineralsalzmediums, das vor der Beimpfung schon Hefeextrakt enthielt mit Bactotrypton oder Pepton, sowie die Zudosierung dieser komplexen Substrate während der Fermentation führte zu keiner weiteren Erhöhung der Biomassekonzentration (Bailey et al. 1987; Tsai et al. 1987). Sehr hohe Biomassekonzentrationen von 80 bis 105 g X/l (Eppstein et al. 1989) wurden durch Kombination der oben beschriebenen Fed-batch-Technik (Dosierung von Glukose und Salzen) durch Begasung mit sauerstoffangereicherter Luft erzielt. Krüger (1989) erzielte durch Begasung mit reinem Sauerstoff 112 g X/l.

Die oben beschriebenen Verfahren sind alle mit dem Nachteil behaftet, daß sie sehr komplexe Nachfütterungs-Systeme darstellen. Die Zugabe aller zusätzlichen Nährstoffe außer Glukose oder mit Magnesiumsulfat versetzter Glukose und Ammoniak bedingen spezielle Nährsubstratdosierungen, die entweder nur zeitweise Zugaben oder zeitgesteuerte Zugabephasen beinhalten. Dies betrifft auch den Einsatz des gasförmigen Substrates Sauerstoff. Anzustreben ist ein Verfahren zur Erzielung hoher Zelldichten unter Verwendung eines Glukose-Mineralsalzmediums, ohne daß außer der üblichen mit Magnesiumsulfat versetzten Glukoselösung und Ammoniak weitere Nährstoffe bei üblicher Begasung mit Luft erforderlich sind, möglichst ohne die Notwendigkeit der Anreicherung mit reinem Sauerstoff.

E. coli Zellen, die als Wirte für Vektoren dienen und somit zur Bildung rekombinanter DNA-Produkte verwendet werden, müssen dem einzusetzenden Expressionssytem für das rekombinante Produkt adäquat fermentiert werden. Dies bedeutet, daß E. coli-Wirte mit anschaltbaren Expressionssystemen zunächst zu hohen Zelldichten fermentiert werden und dann die Anschaltung der rekombinanten DNA-Expression vorgenommen wird. Für E. coli sind auch eine Reihe homologer und heterologer Expressionssysteme, die konstitutiv sind bzw. bei denen die Expression bei niederen spezifischen Wachstumsraten höher ist als bei der im jeweiligen Nährmedium maximal möglichen spezifischen Wachstumsrate $\mu_{max}$, bekannt. Die spezifische Wachstumsrate ist wie folgt definiert:

$\mu = 1/X \cdot dX/dt$.

Es sind einige Lösungen bekannt, mit denen ein Wachstum von E. coli mit $\mu < \mu_{max}$ realisiert wurde. So erreichten Zabriskie und Arcuri (1986) durch die Zufütterung der Kohlenstoffquelle zur Fermentationslösung mit einer konstanten Rate, die geringer war als diejenige, die zu $\mu_{max}$ geführt hätte, daß die Kultur mit $\mu < \mu_{max}$ wuchs. Nachteil dieser Vorgehensweise ist jedoch, daß sich $\mu$ ständig ändert. Bei Konstanthaltung der Dosierungsrate über der gesamten Fed-batch-Prozess würde $\mu$ ständig fallen.

Allen und Luli (1985) entwickelten eine Strategie, mit der die spezifische Wachstumsrate $\mu$ mit zunehmender Zelldichte von Zeit zu Zeit durch Einstellung neuer Zufütterungsraten der Kohlenstoffquelle zeitweise neu eingestellt wurde. Diese Strategie impliziert den Nachteil, daß sich $\mu$ in einem Toleranzbereich, der von der Häufigkeit der off-line Messungen von Glukose und Zelldichte abhängt, ständig ändert, obwohl ein Wachstum mit $\mu < \mu_{max}$ erzwungen wird. Änderungen in der spezifischen Wachstumsrate ziehen Unstetigkeiten im Stoffwechsel nach sich und können destabilisierend wirken, insbesondere dann, wenn es zur Bildung von inhibitorisch wirkenden Nebenprodukten des Metabolismus kommt. Um ein Wachstum mit konstantem $\mu$ zu realisieren, wurde von Lee und Mohler (1989) eine kontrollierte Wachstumsraten-Fermentation vorgestellt. Kernstück der Vorgehensweise ist die ständige Messung des von den Zellen während des Wachstums gebildeten Kohlendioxides, die hieraus per Computer berechnete jeweils aktuelle Wachstumsrate und die sofortige Realisierung der zur Aufrechterhaltung der Sollwert-Wachstumsrate notwendigen Einstellung der Dosierrate der Kohlenstoffquelle mit der gekoppelten Zufütterung der C-Quelle. Auf diese Weise gelang Lee und Mohler (1989) die Kultivierung von E. coli mit annähernd konstantem $\mu < \mu_{max}$ im Bereich der Zelldichten von 0.3 bis 60 g X/l ( EP 0315944 A2). Die Strategie der Konstanthaltung von $\mu$ über die Meßgröße $CO_2$ beinhaltet aber prinzipiell den Nachteil, daß $CO_2$ nicht in allen Fällen ein wachstumskorreliertes Signal ist. Dies trifft vor allem dann zu, wenn Umstellungen im Stoffwechsel stattfinden oder anaplerotische Biosynthesewege von den Zellen genutzt werden, was besonders bei sehr hohen Zelldichten und Kohlenstoffmangel auftreten kann. Ein weiterer Nachteil der Vorgehensweise von Lee und Mohler besteht darin, daß nach Ausschöpfung aller Möglichkeiten zur Erhöhung des Sauerstoffeintrages im jeweils verwendeten Fermentorsystem das Wachstum schnell abbricht, da die Gelöstsauerstoffkonzentration sofort abfällt. Nach der Wachstumsphase mit $\mu = const < \mu_{max}$ ist somit eine spürbare Verlängerung des Wachstums zur weiteren Steigerung der Zelldichte mit $\mu < \mu_{max}$ bei ständig fallenden $\mu$ nicht möglich. Desweiteren ist die Gelöstkohlendioxidkonzentration im eingestellten pH-Bereich sehr stark vom pH-Wert abhängig; kleine pH-Änderungen wirken sich negativ auf das Meßsignal aus und können zu einer Verfalschung der $\mu$-Berechnung führen.

Deshalb soll auch Inhalt dieser Erfindung sein, ein Fermentationsverfahren zu beschreiben, mit dem man E. coli gezielt mit stabilem Stoffwechsel zu sehr hohen Zelldichten führen kann.

## Ziel der Erfindung

Das Ziel der Erfindung besteht in der Beschreibung eines Verfahrens zur Erzielung hoher Zelldichten von E. coli in einem Rührkesselfermentor, wobei nach einem Wachstum mit maximaler spezifischer Wachstumsrate ein Wachstum mit submaximaler spezifischer Wachstumsrate erzwungen und die Gelöstsauerstoffkonzentration im Fermentationsmedium auf oder oberhalb eines definierten Wertes gehalten wird.

## Darlegung des Wesens der Erfindung

Der Erfindung liegt die Aufgabe zugrunde, unter Vermeidung der Nachteile der bisher bekannten Lösungen ein Verfahren zu beschreiben, nach dem E. coli in einem Glukose-Mineralsalzmedium in einem Rührkesselfermentor zu hohen Zelldichten fermentiert werden kann, ohne daß außer der Nachfütterung von mit Magnesiumsulfat supplementierter Glukose, Ammoniak und Antischaummittel keine weiteren Dosierungen von anderen Nährsubstraten erforderlich sind, und ohne daß zur Begasung die Anreicherung der Zuluft mit Sauerstoff notwendig ist. Dabei sollte so fermentiert werden, daß das Wachstum der E. coli Zellen zunächst mit maximaler spezifischer Wachstumsrate $\mu_{max}$ und danach in einem angemessenen Zeitraum mit submaximaler

spezifischer Wachstumsrate erfolgt.

Die der Erfindung zugrundeliegende Aufgabe wird durch ein Verfahren zur Hochzelldichte-Fermentation von Escherichia coli im Rührkesselfermentor gelöst, bei dem man in einem Medium mit einem Gehalt an Stickstoffquelle, organischer Kohlenstoffquelle und Mineralsalzen fermentiert und die Fermentation abschnittweise durchführt, indem man

- zuerst einen Batch-Abschnitt mit maximaler spezifischer Wachstumsrate und

  danach einen Fed-Batch-Abschnitt mit submaximaler spezifischer Wachstumsrate vorsieht und

  bei diesem Fed-Batch-Abschnitt die Gelöstsauerstoffkonzentration im Fermentationsmedium auf oder oberhalb eines definierten Wertes hält,

  dadurch **gekennzeichnet**, daß man einen Fed-Batch-Abschnitt mit submaximaler spezifischer Wachstumsrate mit einem etwa konstanten zeitlichen Profil vorsieht, dadurch realisiert, daß man unter Verwendung einer mit $MgSO_4$ supplementierten Glukoselösung entweder einen vorgegebenen pO2-Wert ≧ 1 % über die Glukosedosierung regelt, und die submaximale Wachstumsrate unter Verwendung der Gleichung

$$\mu(t) = \frac{Q_{O2}(t)}{K + \int_{t_0}^{t} Q_{O2}(\tau)d\tau}$$

  wobei:

  $Q_{O2}$ die Sauerstoffverbrauchsrate und

  K der Quotient aus der Biotrockenmassekonzentration zum Zeitpunkt $t_0$ und dem Ertragskoeffizienten für Sauerstoff ist.

  vorsieht, oder

  eine gesteuerte Glukosedosierung durchgeführt wird, wobei der pO2-Wert auf einen konstanten Wert ≧ 1 % eingestellt wird, und während der gesamten Fermentation der gasförmige Sauerstoff nur mit Hilfe von Luft zugeführt wird.

Zur Durchführung des erfindungsgemäßen Verfahrens kann man E. coli K12 (ATCC 10 798) oder einen Abkömmling davon und vorzugsweise den Abkömmling E. coli TG1 (DSM 6 056) fermentieren.

Man kann die Fermentation in Gegenwart von Supplinen vornehmen und dadurch Auxotrophien kompensieren. Liegt eine Thiamin-Auxotrophie vor, so kann man den E.-coli-Stamm in Gegenwart von Thiamin fermentieren. Beispielsweise kann man E. coli TG1 in Gegenwart einer Thiamin-Konzentration ≦ 4,5 mg/l fermentieren.

Als organische Kohlenstoffquelle kann man zusätzlich zur Glukose auch Saccharose, Lactose und/oder Glycerin verwenden. Gemäß einer speziellen Ausführungsform kann man im Fed-Batch-Abschnitt eine mit Magnesiumsulfat supplementierte Glukoselösung verwenden, die ein Antischaummittel umfaßt.

Gemäß einer weiteren speziellen Ausführungsform des erfindungsgemäßen Verfahrens kann man im Fed-Batch-Abschnitt mit Magnesiumsulfat einer Konzentration ≦ 19,2 g $MgSO_4 \cdot 7H_2O$/l supplementierte Glukoselösung einer Konzentration ≦ 700 g Glukose/l verwenden.

Gemäß einer weiteren speziellen Ausführungsform kann das in das erfindungsgemäße Verfahren eingesetzte Nährmedium bereits alle Nährsubstrate für die gesamte Fermentation mit den Ausnahmen enthalten, daß

- wässrige Ammoniaklösung einer Konzentration ≦ 25 % zur pH-Einstellung zudosiert wird,
- im Fed-Batch-Abschnitt mit Magnesiumsulfat einer Konzentration ≦ 19,2 g $MgSO_4 \cdot 7H_2O$/l supplementierte Glukoselösung einer Konzentration ≦ 700 g Glukose/l zudosiert wird und
- gegebenenfalls Antischaummittel, beispielsweise Ucolub N115, zudosiert wird.

Das in das erfindungsgemäße Verfahren eingesetzte Nährmedium kann qualitativ folgende Komponenten umfassen: Glukose, Kaliumdihydrogenphosphat, Diammoniumhydrogenphosphat, Magnesiumsulfat, Eisenzitrat, Kobaltchlorid, Manganchlorid, Kupferchlorid, Borsäure, Natriummolybdat, Zinkacetat, Titriplex III und/oder Zitronensäure sowie ggf. Antischaummittel und ggf. Suppline zur Kompensation von Auxotrophien.

Insbesondere kann das eingesetzte Nährmedium beispielsweise folgende Komponenten umfassen: Glukose (≦ 50 g/l), $KH_2PO_4$ (≦ 13,3 g/l), $(NH_4)_2HPO_4$ (≦ 4 g/l), $MgSO_4 \cdot 7H_2O$ (≦ 1,2 g/l), Eisenzitrat (≦ 60 mg/l), $CoCl_2 \cdot 6H_2O$ (≦ 2,5 mg/l), $MnCl_2 \cdot 4H_2O$ (≦ 15 mg/l), $CuCl_2 \cdot 2H_2O$ (≦ 1,5 mg/l), $H_3BO_3$ (≦ 3 mg/l), $Na_2MoO_4 \cdot 2H_2O$ (≦ 2,5 mg/l), $Zn(CH_3COO)_2 \cdot 2H_2O$ (≦ 8 mg/l), Titriplex III (≦ 8,4 mg/l) und/oder Zitronensäure (≦ 1,7 g/l) sowie ggf. Antischaummittel Ucolub N115 (≦ 0,1 ml/l).

Zur Durchführung des erfindungsgemäßen Verfahrens kann man die Bestandteile des Nährmediums in folgender Reihenfolge in den Fermentor einbringen:

- zuerst Kaliumdihydrogenphosphat, Diammoniumhydrogenphosphat und/oder Zitronensäure;

- danach eine gegebenenfalls aus Stammlösungen angesetzte Lösung von Kobaltchlorid, Manganchlorid, Kupferchlorid, Borsäure, Natriummolybdat, Zinkacetat und/oder Titriplex III,
- danach Eisenzitrat,
- danach gegebenenfalls ein Antischaummittel, wonach man sterilisiert.

Für die aus Stammlösungen angesetzte Lösung kann man zuerst Titriplex III vorlegen.

Nach Sterilisation der im Fermentor vorgelegten Lösung kann man eine sterilisierte Lösung von Glukose und/oder eine sterilisierte Lösung von Magensiumsulfat in den Fermentor einbringen.

Nach der Beimpfung des Nährmediums kann vor dem Batch-Abschnitt eine lag-Phase auftreten.

Im Batch-Abschnitt kann man bei einem pH $\leqq$ 7,5, insbesondere im Bereich von 6,6 bis 6,9 und vorzugsweise bei etwa 6,8 fermentieren. Die Einstellung des pH-Wertes kann mit wässriger Ammoniaklösung (einer Konzentration von $\leqq$ 25 %) erfolgen.

Gemäß einer speziellen Ausführungsform des erfindungsgemäßen Verfahrens kann man im Batch-Abschnitt durch allmähliche Erhöhung der Rührerdrehzahl einen $pO_2 \geqq 1\%$, vorzugsweise im Bereich von 5 bis 20 % und insbesondere etwa 10 % einstellen.

Nach beendetem Batch-Abschnitt kann man zur submaximalen spezifischen Wachstumsrate des Fed-Batch-Abschnittes dadurch übergehen, daß man

- entweder die Rührerdrehzahl erniedrigt, insbesondere im Bereich von 5 bis 60 min,
- oder die Rührerdrehzahl konstant hält, insbesondere im Bereich von 0,5 bis 10 h.

Im Fed-Batch-Abschnitt kann man mit Hilfe der Glukosedosierung einen $pO_2$ im Bereich von 5 bis 20 % und insbesondere von etwa 10 % einstellen.

Auch kann man im Fed-Batch-Abschnitt die gewünschte submaximale spezifische Wachstumsrate durch Erhöhen der Rührerdrehzahl, der Begasungsrate und/oder des Druckes einstellen.

Man kann die submaximale spezifische Wachstumsrate mit Hilfe des Sauerstoffgehalts der Fermentorabluft überwachen.

Gemäß einer weiteren Ausführungsform des erfindungsgemäßen Verfahrens kann man im Fed-Batch-Abschnitt, nachdem der technisch maximal zu realisierende Sauerstoffeintrag erreicht worden ist, die Kultur mit Hilfe der $pO_2$-Regelung durch Glukose-Dosierung bei sinkender submaximaler spezifischer Wachstumsrate weiterfermentierten.

Der Vollständigkeit halber sei auch angemerkt, daß man die Konzentration an Biotrockenmasse dadurch variieren kann, daß man

- das Verhältnis der Dauer des Batch-Abschnitts zur Dauer des Fed-Batch-Abschnitts und/oder
- im Fed-Batch-Abschnitt die Höhe der etwa konstant zu haltenden submaximalen spezifischen Wachstumsrate variiert. So können mindestens etwa 95 g X l beispielsweise bei einer Kultivierung von E. coli TG1 bei $\mu$ = 0,107 1/h = const im Fed-Batch-Aschnitt erreicht werden.

Nachdem für das jeweilige Fermentorsystem die Sauerstoffeintragsleistung nicht mehr gesteigert werden kann, erlaubt überraschenderweise das vorliegende Verfahren ein weiteres Wachstum; allerdings bei fallendem $\mu$ ohne eintretenden Mangel an Sauerstoff für die Zellen in der Kulturlösung mittels der vorliegenden $pO_2$-Regelung. In dem oben beschriebenen Medium wurde auf diese Weise mit E. coli TG1 eine Biotrockenmassekonzentration von mindestens 110 g X/l erhalten, wobei das Endvolumen zu 76 % des Fermentornettovolumens nicht überschritten wird.

Nachstehend wird die Erfindung durch eine Figur und ein Ausführungsbeispiel näher erläutert.

**Ausführungsbeispiel**

Das Hochzelldichte-Fermentationsvertahren soll am Beispiel der Kultivierung Von E. coli TG1 im 72l-Fermentor (B.Braun Melsungen AG, Typ 8015.1.01) näher beschrieben werden.

Die zu beimpfende Nährlösung von 37 l enthielt folgende Bestandteile: Glukose (25 g/l), $KH_2PO_4$ (13.3 g/l), $(NH_4)_2HPO_4$ (4 g/l), $MgSO_4 \cdot 7H_2O$ (1.2 g/l), Eisen-(III)-zitrat (60 mg/l), $CoCl_2 \cdot 6H_2O$ (2,5 mg/l), $MnCl_2 \cdot 4H_2O$ (15 mg/l), $CuCl_2 \cdot 2H_2O$ (1.5 mg/l), $H_3BO_3$ (3 mg/l), $Na_2MoO_4 \cdot 2H_2O$ (2.5 mg/l), $Zn(CH_3COO)_2 \cdot 2H_2O$ (8 mg/l), Thiamin (4.5 mg/l), Titriplex III (8.4 mg/l), Zitronensäure (1.7 g/l) und Ucolub N115 (0.1 ml/l). Die Zubereitung dieser Nährlösung wurde folgendermaßen vorgenommen: Zunächst wurden 148 g $(NH4)_2HPO_4$, 492.1 g $KH_2PO_4$ und 62.9 g Zitronensäure als Trockensubstanz in ca. 30 l deionisiertem Wasser im Fermentor gelöst; dann folgten der Zusatz von 257.4 ml Spurengemischlösung, der Zusatz einer Eisen(III)-zitratlösung (2.22 g Eisen(III)-zitrat in 300 ml) und von 3.7 ml Ucolub N115. Die Spurengemischlösung wurde aus Stammlösungen in nachfolgender Weise hergestellt: 62.2 ml Titriplex III (5 g/l), 34.3 ml $CoCl_2 \cdot 2H_2O$ (2.7 g/l), 34.7 ml $CuCl_2 \cdot 2H_2O$ (1.6 g/l), 34.7 ml $MnCl_2 \cdot 4H_2O$ (16 g/l), 27.8 ml $H_3BO_3$ (4 g/l), 30.8 ml $Na_2MoO_4 \cdot 2H_2O$ (3 g/l) und 32.9 ml $Zn(CH_3COO)_2 \cdot 2H_2O$ (9 g/l). Die Lösung im Fermentor wurde auf übliche Weise sterilisiert. Anschließend wurden zur Komplettierung noch separat sterilisierte Glukoseklösung (1.0175 kg Glukosemonohydrat in 2.5 l $H_2O$), sterilisierte Magnesiumsulfatlösung (44.4 g $MgSO_4 \cdot 7H_2O$ in 1 l $H_2O$), sterilfiltrierte Thiaminiö-

sung (166.5 mg in 100 ml $H_2O$) zugesetzt, der pH-Wert mit wäßriger Ammoniaklösung (25%) auf 6.8 eingestellt, und schließlich mit sterilem $H_2O$ bis zum Erreichen von 36.8 l aufgefüllt. Anschließend erfolgte die Beimpfung mit 200 ml einer aufgetauten Glycerin-Kulturkonserve (20% v/v) von E. coli TG1.

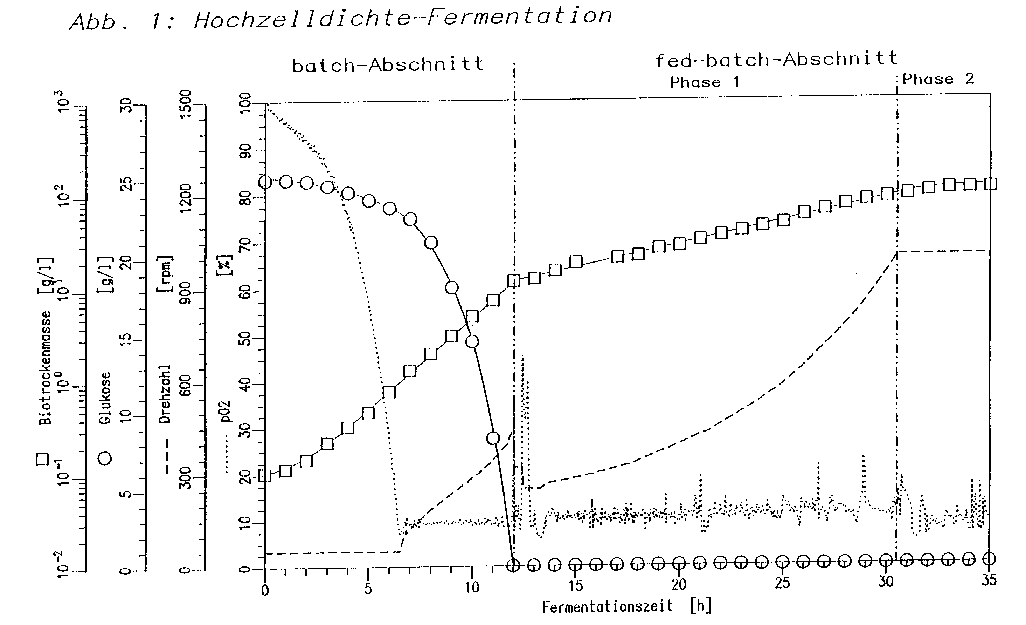

Die Kultivierung von E. coli TG1 wurde bei einer Temperatur von 28 °C, einem Druck von 1.5 bar, einem pH-Wert von 6.8 und einer Begasungsrate von 85 l/min vorgenommen. Der pH-Wert wurde während der gesamten Fermentation durch geregelte Dosierung von 25% $NH_3$ konstant gehalten. In Abb.1 sind die zeitlichen Verläufe der Rührerdrehzahl, der Konzentration der Biotrockenmasse X, der Glukosekonzentration und der Gelöstsauerstoffkonzentration dargestellt.

Im Batch-Abschnitt der Hochzelldichte-Fermentation wuchs die Kultur nach der Beimpfung zum Zeitpunkt t = 0 nach einer kurzen lag-Phase von ca. 2 h mit maximaler spezifischer Wachstumsrate ($\mu_{max}$ = 0.456 1/h). Nach Erreichen des Wertes von 10% wurde der $pO_2$ durch geregelte Erhöhung der Rührerdrehzahl im weiteren Verlauf konstant gehalten. Nach Verbrauch der zu Beginn vorgelegten Glukose war der Batch-Abschnitt beendet. Der $pO_2$ stieg drastisch an. Danach erfolgte keine weitere Regelung des $pO_2$ über die Rührerdrehzahl. Zur Absenkung der spezifischen Wachstumsrate von $\mu_{max}$ = 0.456 auf $\mu$ = 0.11 1/h wurde die Rührerdrehzahl von 420 U/min auf 250 U/min im Zeitraum von 30 Minuten abgesenkt. Anschließend wurde die $pO_2$ - Regelung über die Dosierung von mit Magnesiumsulfat (19.2 g $MgSO_4 \cdot 7H_2O$ /l) supplementierter Glukoselösung (700 g/l) in Betrieb genommen. Die Konstanthaltung von $\mu$ in der Phase 1 des Fed-batch-Abschnittes erfolgte durch Erhöhung des Sauerstoffeintrages. Zu diesem Zweck wurde die Drehzahl über einen PI-Regler mit der Regelgröße $\mu$ gestellt. Die spezifische Wachstumsrate wurde indirekt aus der Sauerstoffbilanz des Systems nach folgender Formel ermittelt:

$$\mu(t) = \frac{Q_{O2}(t)}{K + \int_{t_0}^{t} Q_{O2}(\tau)d\tau}$$

wobei:

$Q_{O2}$ die Sauerstoffverbrauchsrate und

K der Quotient aus der Biotrockenmassekonzentration zum Zeitpunkt $t_0$ und dem Ertragskoeffizienten für Sauerstoff ist.

Am Ende der ersten Phase des Fed-batch-Abschnittes wurde eine Konzentration an Biotrockenmasse von 95 g/l erreicht. Aufgrund des nicht mehr erhöhbaren Sauerstoffeintrages in die Kulturlösung nach Erreichen der technisch maximal möglichen Rührerdrehzahl wuchs die Kultur in der Phase 2 des Fed-batch-Abschnittes dann mit ständig fallendem $\mu$. Am Ende dieser Phase und somit zu Fermentationsabschluß konnte eine Biotrockenmasse von 110 g X/l erhalten werden.

**Literatur**


- Allen, B. R. und Luli, G. W. (1985) A gradient-feed process for obtaining high cell densities for recombinant E. coli. Zitiert in Lee und Mohler: EP 0315944 A2
- Bailey, F. J., Blankenship, J., Condra, J.H., Maigetter, R. Z., Ellis, R. W. (1987) High-cell-density fermentation studies of a recombinant E. coli that expresses atrial natriuretic factor. J. Industrial Microbiol. 2: 47-52
- Bauer, S., Shiloach, I. (1974) Maximal exponantial growth rate and yield of E. coli obtainable in a bench-scale fermentor. Biotechn. Bioeng. 16: 933-941
- Bauer, S., White, M. D. (1976) Pilot scale exponential growth of Escherichia coli W to high cell concentration with temperature variation. Biotechn. Bioeng. 18: 839-846
- Bauer, S., Ziv, E. (1976) Dense growth of aerobic bacteria in a bench-scale fermentor. Biotechn. Bioeng. 18: 81-94
- Cutayar, J. M., Poillon, D. (1989) High cell density culture of E. coli in a fed-batch system with dissolved oxygen as substrate feed indicator. Biotechnol. Lett. 11: 155-160
- Eppstein, L., Shevitz, J., Yang, X. M. und Weiss, S. (1989) Increased biomass production in a benchtop fermentor. Bio/Technology 7: 1178-1181
- Fass, R., Clem, T.R., Shiloach, J. (1989) Use of a noval air separation system in a fed-batch fermentative culture of Escherichia coli. Appl. Environ. Microbiol. 55: 1305-1307
- Fieschko, J., Ritch, T. (1986) Production of human alpha consensus interferon in recombinant Escherichia coli. Chem. Eng. Comm. 45: 229-240
- Gleiser, I. E., Bauer, S. (1981) Growth of E. coli to high cell concentration by oxygen level linked control of carbon source concentration. Biotechn. Bioeng. 23: 1015-1021
- Jung, G., Denefle, P., Becquart, J., Mayaux, J. F. (1988) High cell density fermentation studies of recombinant Escherichia coli strains expressing human interleukin-1$\beta$. Ann. Inst. Pasteur/Microbiol. 139: 129-146

- Krüger, B. (1989) Ausbeutesteigerung in der Fermentation durch Computereinsatz. BioTec Meßtechnik, Okt, 65-67
- Lee, S. und Mohler, R. D. (1989) Controlled growth rate fermentation. EP 0315944 A2
- Mizutani, S., Mori, H., Shimizu, S., Sakaguchi, K., Kobayashi, T. (1986) Effect of amino acid supplement on cell yield and gene product in Escherichia coli harboring plasmid. Biotechn. Bioeng. 28: 204-209
- Mori, H., Yano, T., Kodayashi, T., Shimizu, S. (1979) High density cultivation of biomass in fed-batch system with DO-stat. J. Chem. Eng. 12: 313-319
- Pan, J. G., Rhee, J. S., Lebeault, J. M.(1987) Physiological contraints in increasing biomass concentration of Escherichia coli B in fed-batch culture. Biotechnol. Lett. 9: 89-94
- Reiling, H. E., Laurila, H., Fiechter, A. (1985) Mass culture of Escherichia coli: Medium development for low and high density cultivation of Escherichia coli B/r in minimal and complex media. J. Biotechnol. 2: 191-206
- Shiloach, J., Bauer, S. (1975) High-yield growth of E. coli at different temperature in a bench scale fermentor. Biotechn. Bioeng. 17: 227-239
- Tsai, L. B., Mann, M., Morris, F., Rotgers, C., Fenton, D. (1987) The effect of organic nitrogen and glucose on the production of recombinant human insulin-like growth factor in high cell density Escherichia coli fermentations. J. Industrial Microbiol. 2: 181-187
- Zabriskie, D. W. und Arcuri, E. J. (1986) Factors influencing productivity of fermentations employing recombinant microorganisms. Enzyme Microb. Technol. 8: 706-717


## Patentansprüche

**1.** Verfahren zur Hochzelldichte-Fermentation von E. coli in einem Rührkesselfermentor, bei dem man in einem Medium mit einem Gehalt an Stickstoffquelle, organischer Kohlenstoffquelle und Mineralsalzen fermentiert und die Fermentation abschnittweise durchführt, indem man
zuerst einen Batch-Abschnitt mit maximaler spezifischer Wachstumsrate und
danach einen Fed-Batch-Abschnitt mit submaximaler spezifischer Waschtumsrate vorsieht und
bei diesem Fed-Batch-Abschnitt die Gelöstsauerstoffkonzentration im Fermentationsmedium auf oder oberhalb eines definierten Wertes hält,
dadurch **gekennzeichnet**, daß man einen Fed-Batch-Abschnitt mit submaximaler spezifischer Wachstumsrate mit einem etwa konstanten zeitlichen Profil vorsieht, dadurch realisiert, daß man unter Verwendung einer mit $MgSO_4$ supplementierten Glukoselösung entweder einen vorgegebenen pO2-Wert ≧ 1 % über die Glukosedosierung regelt, und die submaximale Wachstumsrate unter Verwendung der Gleichung

$$\mu(t) = \frac{Q_{O2}(t)}{K + \int_{t_0}^{t} Q_{O2}(\tau)d\tau}$$

wobei:
$Q_{O2}$ die Sauerstoffverbrauchsrate und
K der Quotient aus der Biotrockenmassekonzentration zum Zeitpunkt $t_0$ und dem Ertragskoeffizienten für Sauerstoff ist.
vorsieht, oder
eine gesteuerte Glukosedosierung durchgeführt wird, wobei der pO2-Wert auf einen konstanten Wert ≧ 1 % eingestellt wird, und
während der gesamten Fermentation der gasförmige Sauerstoff nur mit Hilfe von Luft zugeführt wird.

**2.** Verfahren nach Anspruch 1, dadurch **gekennzeichnet**, daß man E. coli K12 (ATCC 10 798) oder einen Abkömmling davon und vorzugsweise den Abkömmling E. coli TG1 (DSM 6 056) fermentiert.

**3.** Verfahren nach einem der vorhergehenden Ansprüche, dadurch **gekennzeichnet**, daß man in Gegenwart von Supplinen fermentiert und Auxotrophien kompensiert.

**4.** Verfahren nach Anspruch 3, dadurch **gekennzeichnet**, daß man bei Thiamin-Auxotrophie des zu fermentierenden E.-coli-Stammes in Gegenwart von Thiamin fermentiert.

**5.** Verfahren nach Anspruch 4, dadurch **gekennzeichnet**, daß man E. coli TG1 in Gegenwart einer Thiamin-Konzentration ≦ 4,5 mg/l fermentiert.

**6.** Verfahren nach einem der vorhergehenden Ansprüche, dadurch **gekennzeichnet**, daß man als organische Kohlenstoffquelle zusätzlich Saccharose, Lactose und/oder Glycerin verwendet.

**7.** Verfahren nach einem der vorhergehenden Ansprüche, dadurch **gekennzeichnet**, daß man

im Fed-Batch-Abschnitt eine mit Magnesiumsulfat supplementierte Glukoselösung verwendet, die ein Antischaummittel umfaßt.

**8.** Verfahren nach einem der vorhergehenden Ansprüche, dadurch **gekennzeichnet**, daß man im Fed-Batch-Abschnitt mit Magnesiumsulfat einer Konzentration ≦ 19,2 g $MgSO_4 \cdot 7H_2O$/l supplementierte Glukoselösung einer Konzentration ≦ 700 g Glukose/l verwendet.

**9.** Verfahren nach einem der vorhergehenden Ansprüche, dadurch **gekennzeichnet**, daß man in dem Verfahren ein Nährmedium einsetzt, das bereits alle Nährsubstrate für die gesamte Fermentation mit den Ausnahmen enthält, daß

- wässrige Ammoniaklösung ≦ 25 % zur pH-Einstellung zudosiert wird,
- im Fed-Batch-Abschnitt mit Magnesiumsulfat einer Konzentration ≦ 19,2 g $MgSO_4 \cdot 7H_2O$/l supplementierte Glukoselösung einer Konzentration ≦ 700 g Glukose/l zudosiert wird und
- gegebenenfalls Antischaummittel zudosiert wird.

**10.** Verfahren nach einem der vorhergehenden Ansprüche, dadurch **gekennzeichnet**, daß man ein Nährmedium einsetzt, das qualitativ folgende Komponenten umfaßt: Glukose, Kaliumdihydrogenphosphat, Diammoniumhydrogenphosphat, Magnesiumsulfat, Eisenzitrat, Kobaltchlorid, Manganchlorid, Kupferchlorid, Borsäure, Natriummolybdat, Zinkacetat, Titriplex III und/oder Zitronensäure sowie gegebenenfalls Antischaummittel und gegebenenfalls Suppline zur Kompensation von Auxotrophien.

**11.** Verfahren nach einem der vorhergehenden Ansprüche, dadurch **gekennzeichnet**, daß man ein Nährmedium verwendet, das folgende Komponenten umfaßt: Glukose (≦ 50 g/l), $KH_2PO_4$ (≦ 13,3 g/l), $(NH_4)_2HPO_4$ (≦ 4 g/l), $MgSO_4 \cdot 7H_2O$ (≦ 1,2 g/l), Eisenzitrat (≦ 60 mg/l), $CoCl_2 \cdot 6H_2O$ (≦ 2,5 mg/l), $MnCl_2 \cdot 4H_2O$ (≦ 15 mg/l), $CuCl_2 \cdot 2H_2O$ (≦ 1,5 mg/l), $H_3BO_3$ (≦ 3 mg/l), $Na_2MoO_4 \cdot 2H_2O$ (≦ 2,5 mg/l), $Zn(CH_3COO)_2 \cdot 2H_2O$ (≦ 8 mg/l), Titriplex III (≦ 8,4 mg/l) und/oder Zitronensäure (≦ 1,7 g/l) sowie gegebenenfalls Antischaummittel (≦ 0,1 ml/l).

**12.** Verfahren nach einem der vorhergehenden Ansprüche, dadurch **gekennzeichnet**, daß man die Bestandteile des Nährmediums in folgender Reihenfolge in den Fermentor einbringt:

- zuerst Kaliumdihydrogenphosphat, Diammoniumhydrogenphosphat und/oder Zitronensäure;
- danach eine gegebenenfalls aus Stammlösungen angesetzte Lösung aus Kobaltchlorid, Manganchlorid, Kupferchlorid, Borsäure, Natriummolybdat, Zinkacetat und/oder Titriplex III,
- danach Eisenzitrat,
- danach gegebenenfalls ein Antischaummittel und danach sterilisiert.

**13.** Verfahren nach Anspruch 12, dadurch **gekennzeichnet**, daß man für die aus Stammlösungen angesetzte Lösung zuerst Titriplex III vorlegt.

**14.** Verfahren nach einem der vorhergehenden Ansprüche, dadurch **gekennzeichnet**, daß man nach Sterilisation der im Fermentor vorgelegten Lösung eine sterilisierte Lösung von Glukose und eine sterilisierte Lösung von Magensiumsulfat in den Fermentor einbringt.

**15.** Verfahren nach einem der vorhergehenden Ansprüche, dadurch **gekennzeichnet**, daß man im Batch-Abschnitt bei einem pH ≦ 7,5, insbesondere im Bereich von 6,6 bis 6,9 und vorzugsweise bei etwa 6,8 fermentiert.

**16.** Verfahren nach Anspruch 15, dadurch **gekennzeichnet**, daß man den pH mit wässriger Ammoniaklösung (≦ 25 %) einstellt.

**17.** Verfahren nach einem der vorhergehenden Ansprüche, dadurch **gekennzeichnet**, daß man im Batch-Abschnitt mit Hilfe allmählicher Erhöhung der Rührerdrehzahl einen $pO_2$ ≧ 1%, vorzugsweise im Bereich von 5 bis 20 % und insbesondere etwa 10 % einstellt.

**18.** Verfahren nach einem der vorhergehenden Ansprüche, dadurch **gekennzeichnet**, daß man nach beendetem Batch-Abschnit dadurch zur submaximalen spezifischen Wachstumsrate des Fed-Batch-Abschnittes übergeht, daß man

- entweder die Rührerdrehzahl erniedrigt, insbesondere im Zeitbereich von 5 bis 60 min,
- oder die Rührerdrehzahl konstant hält, insbesondere im Zeitbereich von 0,5 bis 10 h.

**19.** Verfahren nach einem der vorhergehenden Ansprüche, dadurch **gekennzeichnet**, daß man im Fed-Batch-Abschnitt mit Hilfe der Glukosedosierung einen $pO_2$ im Bereich von 5 bis 20 % und insbesondere von etwa 10 % einstellt.

**20.** Verfahren nach einem der vorhergehenden Ansprüche, dadurch **gekennzeichnet**, daß man im Fed-Batch-Abschnitt durch Erhöhung der Rührerdrehzahl, der Begasungsrate und/oder des Druckes den adequaten Sauerstoffeintrag einstellt.

**21.** Verfahren nach einem der vorhergehenden Ansprüche, dadurch **gekennzeichnet**, daß man die submaximale spezifische Wachstumsrate mit Hilfe des Sauerstoffgehalts der Fermenterabluft überwacht.

**22.** Verfahren nach einem der vorhergehenden Ansprüche, dadurch **gekennzeichnet**, daß man im Fed-Batch-Abschnitt, nachdem der technisch maximal zu realisierende Sauerstoffeintrag erreicht worden ist, die Kultur mit Hilfe der $pO_2$-Regelung durch Glukose-Dosierung bei sinkender submaximaler spezifischer Wachstumsrate weiterfermentiert.

**23.** Verfahren nach einem der vorhergehenden Ansprüche, dadurch **gekennzeichnet**, daß man die Konzentration an Biotrockenmasse variiert, indem man

- das Verhältnis der Dauer des Batch-Abschnitts zur Dauer des Fed-Batch-Abschnitts und/oder
- im Fed-Batch-Abschnitt die Höhe der konstant zu haltenden submaximalen spezifischen Wachstumsrate variiert.

**Claims**

**1.** Process for the high cell-density fermentation of E. coli in an agitated tank fermenter, wherein fermentation is carried out in a medium containing a nitrogen source, organic carbon source and mineral salts, and the fermentation is carried out in sections by providing

firstly a batch section with maximum specific growth rate and

then a fed-batch section with submaximum specific growth rate, and

in this fed-batch section the dissolved oxygen concentration in the fermentation medium is kept at or above a defined value,

characterised in that a fed-batch section with submaximum specific growth rate with an approximately constant time profile is provided, achieved by controlling, with use of a glucose solution supplemented with $MgSO_4$, either a predetermined pO2 ≧ 1% via the glucose metering, and the submaxiumum growth rate is provided with use of the equation

$$\mu(t) = \frac{Q_{O_2}(t)}{K + \int_{t_0}^{t} Q_{O_2}(\tau)\,d\tau}$$

where:

$Q_{O_2}$ is the oxygen consumption rate and

K is the quotient of the dry biomass concentration at time $t_0$ and the yield coefficient for oxygen.

or a controlled glucose metering is carried out, the pO2 being adjusted to a constant value ≧ 1%, and

the gaseous oxygen is introduced only with the aid of air throughout the fermentation.

**2.** Process according to Claim 1, characterised in that E. coli K12 (ATCC 10 798) or a derivative thereof and, preferably, the derivative E. coli TG1 (DSM 6 056) is fermented.

**3.** Process according to either of the preceding claims, characterised in that fermentation is carried out in the presence of supplines, and auxotrophies are compensated.

**4.** Process according to Claim 3, characterised in that, in the case of thiamine auxotrophy of the E. coli strain to be fermented, fermentation is carried out in the presence of thiamine.

**5.** Process according to Claim 4, characterised in that E. coli TG1 is fermented in the presence of a thiamine concentration ≦ 4.5 mg/l.

**6.** Process according to any of the preceding claims, characterised in that sucrose, lactose and/or glycerol is additionally used as organic carbon source.

**7.** Process according to any of the preceding claims, characterised in that a glucose solution which is supplemented with magnesium sulphate and which contains an antifoam agent is used in the fed-batch section.

**8.** Process according to any of the preceding claims, characterised in that glucose solution of a concentration ≦ 700 g of glucose/l supplemented with magnesium sulphate of a concentration ≦ 19.2 g of $MgSO_4.7H_2O$/l is used in the fed-batch section.

**9.** Process according to any of the preceding claims, characterised in that the nutrient medium employed in the process already contains all the nutrient substrates for the entire fermentation with the exceptions that

- aqueous ammonia solution ≦ 25% is metered in for pH adjustment,
- glucose solution of a concentration ≦ 700 g of glucose/l supplemented with magnesium sulphate of a concentration ≦ 19.2 g of $MgSO_4.7H_2O$/l is metered in during the fed-batch section and
- where appropriate antifoam agent is metered in.

**10.** Process according to any of the preceding claims, characterised in that the nutrient medium employed qualitatively comprises the following components: glucose, potassium dihydrogen phosphate, diammonium hydrogen phosphate, magnesium sulphate, iron citrate, cobalt chloride, manganese chloride, copper chloride, boric acid, sodium molybdate, zinc acetate, Titriplex III and/or citric acid and, where appropriate, antifoam agents and, where appropriate, supplines to compensate auxotrophies.

**11.** Process according to any of the preceding claims, characterised in that the nutrient medium used comprises the following components: glucose (≦ 50 g/l), $KH_2PO_4$ (≦ 13.3 g/l), $(NH_4)_2HPO_4$ (≦ 4 g/l), $MgSO_4.7H_2O$ (≦ 1.2 g/l), iron citrate (≦ 60 mg/l), $CoCl_2.6H_2O$ (≦ 2.5 mg/l), $MnCl_2.4H_2O$ (≦ 15 mg/l), $CuCl_2.2H_2O$ (≦ 1.5 mg/l), $H_3BO_3$ (≦ 3 mg/l), $Na_2MoO_4.2H_2O$ (≦ 2.5 mg/l), $Zn(CH_3COO)_2.2H_2O$ (≦ 8 mg/l), Titriplex III (≦ 8.4 mg/l) and/or citric acid (≦ 1.7 g/l) and, where appropriate, antifoam agent (≦ 0.1 ml/l).

**12.** Process according to any of the preceding claims, characterised in that the constituents of the nutrient medium are introduced into the fermenter in the following sequence:

- first potassium dihydrogen phosphate, dimonium hydrogen phosphate and/or citric acid;
- then a solution, which is made up from stock solutions where appropriate, of cobalt chloride, manganese chloride, copper chloride, boric acid, sodium molybdate, zinc acetate and/or Titriplex III,
- then iron citrate,
- then, where appropriate, an antifoam agent and then sterilised.

**13.** Process according to Claim 12, characterised in that Titriplex III is introduced first for the solution made up from stock solutions.

**14.** Process according to any of the preceding claims, characterised in that, after sterilisation of the solution initially introduced into the fermenter, a sterilised solution of glucose and a sterilised solution of magnesium sulphate are introduced into the fermenter.

**15.** Process according to any of the preceding claims, characterised in that fermentation in the batch section is carried out at a pH ≦ 7,5, in particular in the range from 6.6 to 6.9 and preferably at about 6.8.

**16.** Process according to Claim 15, characterised in that the pH is adjusted using aqueous ammonia solution (≦ 25%).

**17.** Process according to any of the preceding claims, characterised in that a $pO_2$ ≧ 1%, preferably in the range from 5 to 20% and, in particular, about 10%, is adjusted in the batch section by gradually increasing the stirrer speed.

**18.** Process according to any of the preceding claims, characterised in that, after the batch section is complete, the transition to the submaximum specific growth rate in the fed-batch section is effected by

- either reducing the stirrer speed, in particular in the time period from 5 to 60 min,
- or keeping the stirrer speed constant, in particular in the time period from 0.5 to 10 h.

**19.** Process according to any of the preceding claims, characterised in that a $pO_2$ in the range from 5 to 20% and, in particulars of about 10% is adjusted in the fed-batch section by means of the glucose metering.

**20.** Process according to any of the preceding claims, characterised in that the appropriate oxygen input is adjusted in the fed-batch section by increasing the stirrer speed, the gas introduction rate and/or the pressure.

**21.** Process according to any of the preceding claims, characterised in that the submaximum specific growth rate is monitored by means of the oxygen content of the air leaving the fermenter.

**22.** Process according to any of the preceding claims, characterised in that after the maximum technically possible oxygen input has been reached in the fed-batch section the culture is fermented further by means of $pO_2$ control by metering in glucose while the submaximum specific growth rate falls.

**23.** Process according to any of the preceding claims, characterised in that the concentration of dry biomass is altered by alteration of
- the ratio of the duration of the batch section to the duration of the fed-batch section and/or
- the level of the submaximum specific growth rate, which is to be kept constant, in the fed-batch section.

**Revendications**

**1.** Procédé permettant la fermentation à haute densité cellulaire d'Escherichia coli dans un appareil de
fermentation formé d'une cuve à agitation, selon lequel on procède à la fermentation dans un milieu contenant une source d'azote, une source organique de carbone et des sels minéraux et on effectue cette fermentation par étapes,

en prévoyant d'abord une étape batch à un taux de croissance spécifique maximal,

puis une étape fed-batch à un taux de croissance spécifique sous-maximal

et en maintenant la concentration de l'oxygène dissous dans le milieu de fermentation, pendant cette étape fed-batch, à une valeur définie ou au-dessus de cette valeur,

caractérisé en ce qu'on prévoit une étape fed-batch à taux de croissance spécifique sous-maximal avec un profil de variation dans le temps qui est pratiquement constant, ce qui est réalisé, en utilisant une solution de glucose additionnée de $MgSO_4$,

soit en réglant une valeur $pO_2$ préfixée ≧ 1% sur toute l'étendue de l'apport dosé de glucose et en prévoyant le taux de croissance sous-maximal en utilisant l'équation

$$\mu(t) = Q_{O2}(t) / (K + \int_{t_0}^{t} Q_{O2}(\tau)d\tau)$$

dans laquelle :

$Q_{O2}$ est le taux de consommation d'oxygène et

K est le quotient de la concentration de la biomasse sèche à l'instant t0 par le coefficient de rendement de l'oxygène,

soit en procédant à un apport dosé de glucose qui est commandé, en réglant la valeur $pO_2$ à une valeur constante ≧ 1%,

et, pendant toute la fermentation, on n'apporte l'oxygène gazeux qu'au moyen d'air.

**2.** Procédé selon la revendication 1, caractérisé en ce qu'on fait fermenter *E. coli* K12 (ATCC 10 798) ou un dérivé de ce dernier et de préférence le dérivé *E. coli* TG1 (DSM 6 056).

**3.** Procédé selon l'une des revendications précédentes, caractérisé en ce qu'on procède à la fermentation en présence de supplines et on compense les auxotrophies.

**4.** Procédé selon la revendication 3, caractérisé en ce que, dans le cas d'une auxotrophie thiamine de la la souche d'*E. coli* à faire fermenter, on procède à la fermentation en présence de thiamine.

**5.** Procédé selon la revendication 4, caractérisé en ce qu'on fait fermenter *E. coli* TG1 en présence d'une concentration de thiamine ≦ 4,5 mg/l.

**6.** Procédé selon l'une des revendications précédentes, caractérisé en ce que, comme source organique de carbone, on utilise en plus du saccharose, du lactose et/ou de la glycérine.

**7.** Procédé selon l'une des revendications précédentes, caractérisé en ce qu'on utilise, dans l'étape fed-batch, une solution de glucose, additionnée de sulfate de magnésium, qui contient un agent antimoussant.

**8.** Procédé selon l'une des revendications précédentes, caractérisé en ce qu'on utilise, dans l'étape fed-batch, une solution de glucose, à une concentration ≦ 700 g de glucose/l, additionnée de sulfate de magnésium à une concentration ≦ 19,2g $MgSO_4 7H_2O$/l.

**9.** Procédé selon l'une des revendications précédentes, selon lequel on utilise dans le procédé un milieu nutritif qui contient déjà tous les substrats nutritifs pour toute la fermentation à l'exception du fait
- qu'on procède à l'apport dosé de la solution aqueuse d'ammoniac à une concentration ≦ 25% pour le réglage du pH,
- que, dans l'étape fed-batch, on procède à l'apport dosé d'une solution de glucose, à une concentration ≦ 700 g de glucose/l, additionnée de sulfate de magnésium à une concentration ≦ 19,2 g $MgSO_4$ $7H_2O$/l., et

- qu'on procède éventuellement à l'apport dosé d'un agent antimoussant.

**10.** Procédé selon l'une des revendications précédentes, caractérisé en ce qu'on utilise un milieu nutritif qui contient, d'une manière qualitative, les composants suivants : glucose, phosphate monopotassique, phosphate diammonique, sulfate de magnésium, citrate de fer, chlorure de cobalt, chlorure de manganèse, chlorure de cuivre, acide borique, molybdate de sodium, acétate de zinc, Titriplex III et/ou acide citrique, ainsi qu'éventuellement agent antimoussant et éventuellement suppline pour compenser les auxotrophies.

**11.** Procédé selon l'une des revendications précédentes, caractérisé en ce qu'on utilise un milieu nutritif qui contient les composants suivants : glucose (≦ 50 g/l), $KH_2PO_4$ (≦ 13,3 g/l), $(NH_4)_2HPO_4$ (≦ 4 g/l), $MgSO_4 . 7H_2O$ (≦ 1,2 g/l), citrate de fer (≦ 60 mg/l), $CoCl_2 . 6H_2O$ (≦ 2,5 mg/l), $MnCl_2 . 4H_2O$ (≦ 15 mg/l), $CuCl_2 . 2H_2O$ (≦ 1,5 mg/l), $H_3BO_3$ (≦ 3 mg/l), $Na_2MoO_4 . 2H_2O$ (≦ 2,5 mg/l), $Zn(CH_3COO)_2 . 2H_2O$ (≦ 8 mg/l), Titriplex III (≦ 8,4 mg/l) et/ou acide citrique (≦ 1,7 g/l), ainsi qu'éventuellement un agent antimoussant (≦ 0,1 ml/l).

**12.** Procédé selon l'une des revendications précédentes, caractérisé en ce qu'on introduit les constituants du milieu nutritif dans l'appareil de fermentation dans l'ordre suivant :

- d'abord le phosphate monopotassique, phosphate diammonique et/ou acide citrique,
- puis une solution de chlorure de cobalt, chlorure de manganèse, chlorure de cuivre, acide borique, molybdate de sodium, acétate de zinc et/ou Titriplex III, éventuellement préparée à partir de liqueurs mères,
- puis le citrate de fer,
- puis éventuellement un agent antimoussant et on stérilise ensuite.

**13.** Procédé selon la revendication 12, caractérisé en ce que, pour la solution préparée à partir de liqueurs mères, on met d'abord le Titriplex III.

**14.** Procédé selon l'une des revendications précédentes, caractérisé en ce qu'après stérilisation de la solution préexistante dans l'appareil de fermentation, on introduit dans cet appareil de fermentation une solution stérilisée de glucose et/ou une solution stérilisée de sulfate de magnésium.

**15.** Procédé selon l'une des revendications précédentes, caractérisé en ce que, dans l'étape batch, on fait fermenter à un pH ≦ 7,5, notamment dans l'intervalle de 6,6 à 6,9 et de préférence à environ 6,8.

**16.** Procédé selon la revendication 15, caractérisé en ce qu'on ajuste le pH au moyen d'une solution aqueuse d'ammoniac (une concentration ≦ 25%).

**17.** Procédé selon l'une des revendications précédentes, caractérisé en ce qu'on ajuste, dans l'étape batch, une $pO_2$ ≧ 1% au moyen d'une augmentation graduelle de la vitesse de rotation de l'agitateur. de préference dans l'intervalle de 5 à 20% et notamment à environ 10%.

**18.** Procédé selon l'une des revendications précédentes, caractérisé en ce qu'une fois l'étape batch terminée, on passe au faux de croissance spécifique sous-maximal de l'étape fed-batch

- soit en abaissant la vitesse de rotation de l'agitateur, notamment dans l'intervalle de 5 à 60 min,
- soit en maintenant constante la vitesse de rotation de l'agitateur, notamment dans l'intervalle de 0,5 à 10 h.

**19.** Procédé selon l'une des revendications précédentes, caractérisé en ce que, dans l'étape fed-batch, on ajuste une $pO_2$ dans l'intervalle de 5 à 20% et notamment à environ 10%, au moyen de l'apport dosé de glucose.

**20.** Procédé selon l'une des revendications précédentes, caractérisé en ce que, dans l'étape fed-batch, on ajuste l'introduction convenable d'oxygène en augmentant la vitesse de rotation de l'agitateur, le taux d'absorption gazeuse et/ou la pression.

**21.** Procédé selon l'une des revendications précédentes, caractérisé en ce qu'on contrôle le taux de croissance spécifique sous-maximal au moyen de la teneur en oxygène de l'air évacué de l'appareil de fermentation.

**22.** Procédé selon l'une des revendications précédentes, caractérisé en ce que, dans l'étape fed-batch et une fois qu'a été atteinte l'introduction d'oxygène maximale qu'il est techniquement possible de réaliser, on poursuit la fermentation de la culture au moyen de la régulation de $pO_2$, par apport dosé de glucose, avec abaissement du taux de croissance spécifique sous-maximal.

**23.** Procédé selon l'une des revendications précédentes, caractérisé en ce qu'on fait varier la concentration de la biomasse séchée

- en faisant varier le rapport de la durée de l'étape batch à la durée de l'étape fed-batch et/ou
- en faisant varier dans l'étape fed-batch le niveau du taux de croissance spécifique sous-maximal qui doit être maintenu constant.

Abb. 1: Hochzelldichte-Fermentation